# EUROPÄISCHE PATENTANMELDUNG

(11) **EP 2 423 661 A1**
(43) Veröffentlichungstag der Anmeldung: **29.02.2012**
(21) Anmeldenummer: 10174557.8
(22) Anmeldetag: 30.08.2010
(51) Int. Cl.: G01N 1/28

(54) **Vorrichtung und Verfahren zum automatisierten Isolieren und Transferieren mindestens einer mikroskopischen Probe von einem Probenträger zu einem Auffangsystem**

(71) Anmelder: Helmholtz Zentrum München Deutsches Forschungszentrum für Gesundheit und Umwelt GmbH, 85764 Neuherberg (DE)
(72) Erfinder: Thalhammer, Stefan, 80639, München (DE)
(74) Vertreter: Peckmann, Ralf

(57) **Zusammenfassung**

Die vorliegende Erfindung betrifft eine Vorrichtung (1) zum automatisierten Isolieren und Transferieren mindestens einer mikroskopischen Probe von einem Probenträger (4) zu einem Auffangsystem (11) für eine automatische, nachgelagerte Analyse, mit: einer Auswähleinrichtung (2) zum Auswählen mindestens einer sich auf dem Probenträger (4) befindlichen Probe; einer ansteuerbaren Isoliereinrichtung (6) zum automatisierten Isolieren der mindestens einen ausgewählten Probe; einer ansteuerbaren Transfereinrichtung (7), welche Trägermittel zum definierten Aufnehmen und definierten Ablegen der mindestens einen ausgewählten, isolierten Probe für einen automatisierten und definierten Transfer der mindestens einen ausgewählten, isolierten Probe von dem Probenträger (4) zu dem Auffangsystem (11) aufweist; und einer Steuereinheit, welche für einen automatisierten Isolier- und Transfervorgang mit der Auswähleinrichtung (2), der Isoliereinrichtung (6) und der Transfereinrichtung (7) datenverbunden ist.

## Beschreibung

Die vorliegende Erfindung bezieht sich auf eine Vorrichtung und ein Verfahren zum automatisierten Isolieren und Transferieren mindestens einer mikroskopischen Probe von einem Probenträger zu einem Auffangsystem.

Obwohl auf beliebige Proben anwendbar, werden die vorliegende Erfindung sowie die ihr zugrundeliegende Problematik in Bezug auf membrangestützte Proben und hier insbesondere auf membrangestützte Mikrodissektate, insbesondere mittels der lasergestützten Mikrodissektion isolierte Mikrodissektate, im Folgenden beispielhaft näher erläutert.

Die lasergestützte Mikrodissektion ermöglicht es, kleinste Bereiche aus einem Gewebeschnitt - dieser kann sowohl fixierte als auch lebende Zellsysteme umfassen - bis hin zu einzelnen Zellen gezielt zu isolieren. Darüber hinaus kann mit der kontaktfreien Lasermanipulation die Mikrodissektion von einzelnen Zellkompartimenten, zum Beispiel Zellorganellen, Chromosomen und Chromosomenfragmenten, gewährleistet werden. Durch eine Kombination von PCR, Klonierungstechniken und Laser-Mikrodissektion ist es möglich, regionspezifische chromosomale Proben für die molekulare Cytogenetik zu entwickeln. Allerdings werden bei diesen Ansätzen nach der Laser-Mikrodissektion die Fragmente mit einer möglichst dünn gezogenen Glasnadel, vergleichbar mit der Glasnadel-Mikrodissektion, aufgesammelt. Eine Weiterentwicklung in der Laser-Mikrodissektion erfolgte in der Einführung der Laser-Capture-Mikrodissektion. Bei diesem Verfahren wird eine Kunststoffmembran auf das zu untersuchende Gebiet aufgebracht. Die zu isolierenden Zellen werden durch lokales Anschmelzen des Films thermisch aus dem Gewebe entfernt.

Einen methodisch unterschiedlichen Ansatz zur Isolierung von Einzelzellen und Zellgruppen stellt das Prinzip des Laser Pressure Catapulting (LPC oder LMPC) dar. Bei dieser Technik wird ein gepulster UV-Laser mit Leistungsspitzen von einigen Watt auf die zu isolierende Geweberegion gerichtet. Das Gewebe ist im Gegensatz zu der Laser-Capture-Mikrodissektion auf einer ultradünnen Trägermembran aufgebracht, welche eine Dicke von wenigen µm aufweist. Mit Hilfe eines gepulsten UV-Mikrolaserstrahls können Zellen oder Zellgruppen gezielt isoliert werden, die in einem weiteren Arbeitsschritt mit Hilfe eines hochenergetischen Lichtpulses in eine Sammelvorrichtung isoliert werden. Nach optischer Kontrolle der Ablage, steht das isolierte Material für weitere biochemische Verfahren zur Verfügung.

In der Druckschrift WO 2005/107949 A1 wird ein Verfahren und eine Vorrichtung zur Erzeugung einer Analyseanordnung mit diskreten, separaten Messbereichen zu biologischen, biochemischen oder chemischen Analysezwecken beschrieben.

In Anbetracht der oben genannten, bekannten Ansätze liegt der vorliegenden Erfindung die Aufgabe zugrunde, eine weiter verbesserte Vorrichtung und ein verbessertes Verfahren bereitzustellen.

Diese Aufgabe wird erfindungsgemäß durch eine Vorrichtung mit den Merkmalen des Patentanspruchs 1 sowie durch ein Verfahren mit den Merkmalen des Patentanspruchs 10 gelöst.

Demgemäß wird eine Vorrichtung zum automatisierten Isolieren und Transferieren mindestens einer mikroskopischen Probe von einem Probenträger zu einem Auffangsystem für eine automatische, nachgelagerte Analyse, mit: einer Auswähleinrichtung zum Auswählen mindestens einer sich auf dem Probenträger befindlichen Probe; einer ansteuerbaren Isoliereinrichtung zum automatisierten Isolieren der mindestens einen ausgewählten Probe; einer ansteuerbaren Transfereinrichtung, welche Trägermittel zum definierten Aufnehmen und definierten Ablegen der mindestens einen ausgewählten, isolierten Probe für einen automatisierten und definierten Transfer der mindestens einen ausgewählten, isolierten Probe von dem Probenträger zu dem Auffangsystem aufweist; und einer Steuereinheit, welche für einen automatisierten Isolier- und Transfervorgang mit der Auswähleinrichtung, der Isoliereinrichtung und der Transfereinrichtung datenverbunden ist; bereitgestellt.

Weiterhin wird ein Verfahren zum automatisierten Isolieren und Transferieren mindestens einer mikroskopischen Probe von einem Probenträger zu einem Auffangsystem, mit folgenden Verfahrensschritten: Auswählen mindestens einer sich auf dem Probenträger befindlichen Probe mittels einer Auswähleinrichtung; automatisiertes Isolieren der mindestens einen ausgewählten Probe mittels einer ansteuerbaren Isoliereinrichtung; automatisiertes und definiertes Transferieren der mindestens einen ausgewählten, isolierten Probe von dem Probenträger zu dem Auffangsystem mittels einer ansteuerbaren Transfereinrichtung, welche Trägermittel zum definierten Aufnehmen und definierten Ablegen der mindestens einen ausgewählten, isolierten Probe aufweist; und Ansteuern der Isoliereinrichtung und der Transfereinrichtung mittels einer mit der Auswähleinrichtung, der Isoliereinrichtung und der Transfereinrichtung datenverbundenen Steuereinheit in Abhängigkeit des Schrittes des Auswählens der mindestens einen Probe; bereitgestellt.

Somit weist die vorliegende Erfindung gegenüber den bekannten Ansätzen den Vorteil auf, dass der Isoliervorgang sowie der gesamte Transferierungsvorgang der isolierten Probe/n von dem Probenträger zu dem Auffangsystem vollständig definiert und steuerbar abläuft. Ferner können vorteilhaft für eine Hochdurchsatz-Analytik alle Prozessschritte synchronisiert und voll automatisiert werden. Weiterhin ermöglicht die Erfindung eine direkt nachgelagerte Analytik des abgelegten Probenmaterials. Noch weiterhin wird eine Beschädigung der isolierten Probe aufgrund des definierten Aufnehmens, Transfers und Ablegens sicher vermieden. Noch weiterhin ist eine optische Kontrolle des eigentlichen Transfervorgangs möglich.

In den Unteransprüchen finden sich vorteilhafte Ausgestaltungen und Verbesserungen der erfindungsgemäßen Vorrichtung und des erfindungsgemäßen Verfahrens.

Unter einem "definierten Aufnehmen", "definierten Ablegen" und "definierten Transfer" der Probe ist vorliegend ein solches Aufnehmen, Ablegen und Transferieren zu verstehen, bei welchem die Position der Probe zu jedem Zeitpunkt von vornherein bekannt und beeinflussbar oder zumindest zu jedem Zeitpunkt von der Steuereinheit ermittelbar ist.

Unter "Trägermittel" ist vorliegend ein Mittel zu verstehen, welches die Proben mechanisch aufnimmt, transferiert und ablegt, d.h. es wird wenigstens zeitweise ein Form-, Stoffund/oder Kraftschluss zwischen der Probe und dem Trägermittel erzeugt.

Gemäß einem bevorzugten Ausführungsbeispiel der vorliegenden Erfindung weist die Auswähleinrichtung eine Mikroskopiereinheit und eine damit gekoppelte Anzeigeeinrichtung, insbesondere einen Touch-Screen, zum Darstellen des mikroskopischen Bildes der auf dem Probenträger zur Verfügung stehenden Proben auf. Vorzugsweise weist die Anzeigeeinrichtung Auswählmittel zum Auswählen der mindestens einen zu isolierenden und zu transferierenden Probe auf, insbesondere unter Zuhilfenahme optischer Markierungsmittel zum graphischen Kennzeichnen der mindestens einen zu isolierenden und zu transferierenden Probe auf dem Touch-Screen. Beispielsweise werden die zu isolierenden Bereiche mittels geometrischer Formen markiert und die derart markierten Bereiche mittels der Isoliereinrichtung entsprechend automatisch herausgetrennt. Eine derartige Auswahl und das dadurch beginnende automatisierte Verfahren ist vorteilhaft leicht zu bedienen. Dadurch wird vorteilhaft eine benutzerfreundliche Vorrichtung sowie ein benutzerfreundliches Verfahren zur Verfügung gestellt.

Gemäß einem weiteren bevorzugten Ausführungsbeispiel weist die Isoliereinrichtung einen geeignet ausgestalteten Laser, insbesondere einen UVa-Laser, zum automatisierten, reproduzierbaren und kontaminationsfreien Isolieren der mindestens einen ausgewählten Probe auf. Dabei dient der Laser lediglich einem definierten Heraustrennen der ausgewählten Probe/n aus dem Gesamtträger und nicht gleichzeitig zum Transferieren der herausgetrennten Probe auf ein entsprechendes Transfersystem.

Nach einer weiteren bevorzugten Ausführungsform weist die Transfereinrichtung ein geeignet ausgestaltetes Unterdrucksaugsystem zum automatisierten, reproduzierbaren und kontaminationsfreien Transfer der mindestens einen zu transferierenden Probe auf. Vorzugsweise weist die Transfereinrichtung ein Autofokussystem zum positionsgenauen Verstellen des Unterdrucksaugsystems in einem vorbestimmten Abstand zu der zu transferierenden Probe auf. Hierdurch wird die Reproduzierbarkeit sowie die Automatisierung des gesamten Transferierungsverfahrens weiter verbessert. Das Unterdrucksystem kann beispielsweise wie in der europäischen Anmeldung mit der Anmeldenummer 08150662 beschrieben ausgebildet sein.

Aufgrund des vorzugsweise verwendeten Unterdrucksaugsystems mit speziellen Trägermitteln wird ein definierter Transport und eine gezielte Ablage von beispielsweise mikrodissektierten Proben auf beispielsweise strukturierten Ablagen in einem Flüssigkeitstropfen, welcher sich insbesondere auf einem Mikrochip oder einer Mikrotiterplatte befindet, ermöglicht. Beispielsweise weist die Transfereinrichtung speziell ausgebildete Trägerarme und daran angebrachte Sauger auf, die einen definierten und reproduzierbaren Transport der Proben gewährleisten. Ferner besteht zu jedem Zeitpunkt des gesamten Isolier- und Transferierungsvorgangs volle Kontrolle über die einzelnen Arbeitsschritte bzw. über den jeweiligen Zustand der isolierten Probe. Ferner ist vorteilhaft auf einfache Weise die Gewinnung von homogenem Probenmaterial für nachgeschaltete Analyseverfahren gewährleistet.

Gemäß einer weiteren bevorzugten Ausführungsform umfasst das Unterdrucksaugsystem mehrere, einzeln ansteuerbare Ansaugeinrichtungen zum gleichzeitigen Transferieren mehrerer Proben. Dadurch kann der Probendurchsatz und somit die Analysemenge weiter vorteilhaft gesteigert werden. Vorstellbar ist hier beispielsweise ein Magazinsystem, welches einzelne Ansaugeinrichtungen aufweist und zunächst eine vorbestimmte Anzahl an Proben sukzessive ansaugt und anschließend diese angesaugte Anzahl an Proben gleichzeitig zu dem zugeordneten Auffangsystem zusammen transferiert und wiederum einzeln und sukzessive ablegt.

Nach einem weiteren bevorzugten Ausführungsbeispiel weist das Auffangsystem einen teilweise geschützten Abschnitt auf, insbesondere einem teilweise geschützten Schlittenschacht, wobei ein angepasster Öffnungsbereich für ein Ablegen der mindestens einen Probe auf dem Auffangsystem durch die Transfereinrichtung vorgesehen ist. Hierdurch wird eine mögliche externe Kontamination vorteilhaft verhindert und ein kontaminationsfreies Transferieren gewährleistet.

Gemäß einer weiteren vorteilhaften Ausführungsform ist eine Feed-Back-Kontrolleinrichtung zum Bestätigen einer erfolgreichen Ablage der mindestens einen Probe auf dem Auffangsystem vorgesehen, welche beispielsweise Bragg-Spiegel derart aufweist, dass sich deren Reflektion nach positiver Ablage der mindestens einen Probe messbar ändert. Dadurch wird vorteilhaft eine Kontrolle einer positiven Ablage gewährleistet, um nachgeschaltete, weitere biochemische Prozesse automatisch und einfach ankoppeln zu können. Beispielsweise wird erst nach einer positiven Rückmeldung durch die Feed-Back-Kontrolleinrichtung zur Bestätigung der positiven Ablage der nächste, nachgeschaltete Prozess gestartet.

Gemäß einer weiteren vorteilhaften Ausführungsform wird die Probe nach einer erfolgereichen Ablage derselben auf dem Auffangsystem automatisch analysiert, wobei die Analyse beispielsweise eine biochemische Analyse, eine optische Analyse, eine PCR-Analyse, eine Nukleinsäure Analyse (DNA, RNA, siRNA, microRNA) und/oder eine Protein Analyse ist.

Die transferierte Probe kann für alle sich anschließenden Verfahren zur qualitativen und quantitativen Nukleinsäure und Protein Diagnostik eingesetzt werden, wie z.B.
1) biochemische Verfahren:
   a. qualitativ: Gelelektrophorese, PCR, 2D Proteingele
   b. quantitativ: RT-PCR (real-time Polymerase Kettenreaktion)
2) optische Verfahren:
   a. qualitativ: Fluoreszenzmikroskopie, Messung der optischen Dichte, ELISA, immunhistologische Verfahren
   b. quantitativ: RT-PCR
3) Nukleinsäureverfahren:
   a. qualitativ: Hybridisierungsverfahren (z.B. FISH, Mikroarraytechniken)
   b. quantitativ: RT-PCR, cGH (vergleichende Genomanalyse, Arraytechnologien)

Durch die Miniaturisierung und der daraus resultierenden erhöhten Prozessdichte können folgende Entwicklungsrichtungen im Bereich der Diagnostik verfolgt werden:
1) Analyse von DNA und Proteinen zur Entwicklung neuer Medikamente oder für den Bereich der personalisierten Medizin
2) Miniaturisierung von Analyseeinheiten in den Nanoliter-Bereich
3) Bestimmung wichtiger Messwerte direkt am Krankenbett (point-of-care Diagnostik, POC).

Die Erfindung wird im Folgenden anhand von Ausführungsbeispielen unter Bezugnahme auf die beiliegenden Figuren der Zeichnung näher erläutert. Von den Figuren zeigen:
- Fig. 1: eine schematische Perspektivansicht einer Vorrichtung zum automatisierten Isolieren und Transferieren einer mikroskopischen Probe von einem Probenträger zu einem Auffangsystem gemäß einem bevorzugten Ausführungsbeispiel der vorliegenden Erfindung in einer Probenentnahmeposition;
- Fig. 2: einen vergrößerten Teilausschnitt aus Fig. 1, wobei insbesondere die Mikroskopiereinrichtung und die Transfereinrichtung dargestellt sind;
- Fig. 3: eine schematische Perspektivansicht der Vorrichtung aus Fig. 1, wobei sich die Transfereinrichtung in einer Probenabgabeposition befindet;
- Fig. 4: eine schematische Perspektivansicht einer Transfereinrichtung sowie eines Auffangsystems gemäß einem weiteren bevorzugten Ausführungsbeispiel der vorliegenden Erfindung; und
- Fig. 5: eine Perspektivansicht der Ansaugeinrichtung der Transfereinrichtung gemäß dem bevorzugten Ausführungsbeispiel aus Fig. 4.

In den Figuren der Zeichnung bezeichnen gleiche Bezugszeichen gleiche oder funktionsgleiche Komponenten, soweit nichts Gegenteiliges angegeben ist.

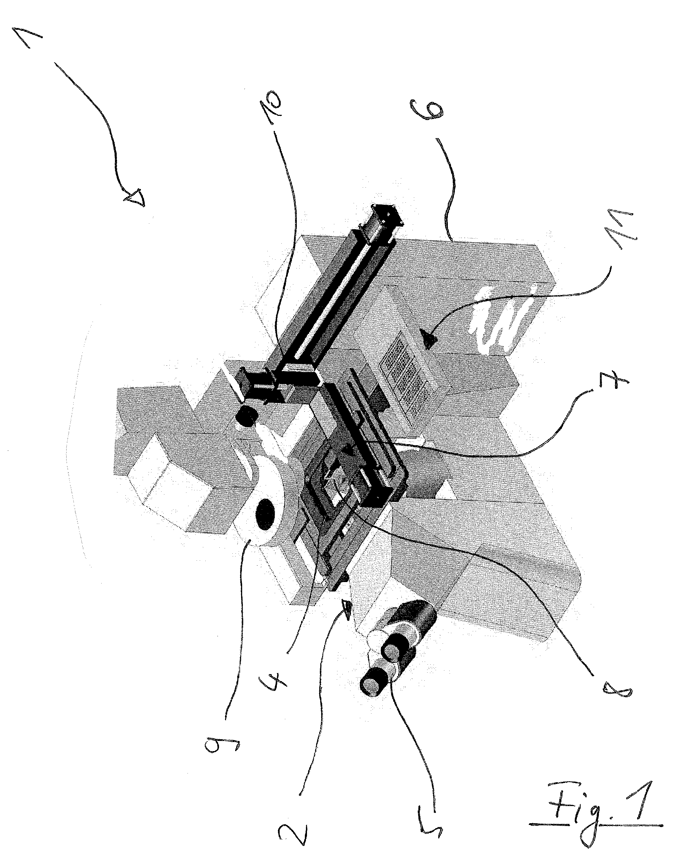

Unter Bezugnahme auf die Fig. 1-3 wird im Folgenden ein erstes bevorzugtes Ausführungsbeispiel der vorliegenden Erfindung näher beschrieben. In Fig. 1 ist eine beispielhafte Vorrichtung 1 zum automatisierten Isolieren und Transferieren einer mikroskopischen Probe perspektivisch dargestellt. Diese beispielhafte Vorrichtung 1 umfasst eine Auswähleinrichtung 2, welche als Bestandteil insbesondere eine Mikroskopiereinheit 3 aufweist, insbesondere in Form eines Objektives. Gemäß einem vorliegenden Ausführungsbeispiel ist ein Objekttisch 4 als Mikroskopbühne ausgebildet und als Bestandteil der Mikroskopiereinheit vorgesehen. Allerdings kann der Objekttisch 4 ebenfalls als unabhängige Mikroskopbühne ausgebildet sein, in welcher ein Objektträger mit beispielsweise Trägermembran einspannbar ist. Weiterhin ist Kondensor 9 vorgesehen, welcher zwischen einer Lichtquelle und der abzubildenden mikroskopischen Probe angeordnet ist. Der Kondensor 9 lenkt das Licht auf die Probe, welches diese durchsetzt und hiernach in das abbildende Objektiv fällt, um eine größtmögliche Auflösung zu erhalten.

An dieser Stelle sei angemerkt, dass die Vorrichtung 1 vorliegend beispielhaft als Mikrodissektionssystem zum Transferieren von isolierten Gewebestücken oder Zellen, im Folgenden als Probe bezeichnet, insbesondere nach einer Laser-Mikrodissektion, ausgebildet ist. Bei einer Laser-Mikrodissektion ist im Allgemeinen die Probe auf einer Trägermembran angeordnet und wird zusammen mit dieser Membran durch eine später detaillierter beschriebene Transfereinrichtung von dem Objekttisch 4 zu einer Analyseeinrichtung transportiert.

Die Auswähleinrichtung 2 umfasst ferner vorzugsweise eine Anzeigeeinrichtung 5, auf welcher das mikroskopische Bild der sich auf dem Objekttisch 4 befindlichen Proben visuell dargestellt wird. Die Anzeigeeinrichtung 5 ist dazu beispielsweise als Touch-Screen und/oder als fernglasartiges Linsensystem wie bei Mikroskopen üblich gemäß Fig. 1 ausgebildet und mit der Mikroskopiereinheit 3 gekoppelt. Mittels einer entsprechenden Software können durch den Benutzer gemäß dem vorliegenden Ausführungsbeispiel vorteilhaft auf einfache und bedienerfreundliche Weise bestimmte, sich auf dem Objekttisch 4 befindliche Proben zur weiteren Bearbeitung, insbesondere Isolierung und Transferierung, markiert werden. Beispielsweise erfolgt eine gezielte Auswahl der zu isolierenden Proben auf dem Objekttisch 4 dadurch, dass das mikroskopische Bild der Proben auf dem Touch-Screen abgebildet wird und die zu isolierenden Bereiche der jeweiligen Proben mittels geometrischer Formen auf dem Touch-Screen markiert werden. Dabei kann lediglich eine zu isolierende Probe oder auch mehrere, gemäß dem vorliegenden Ausführungsbeispiel hintereinander zu isolierende und zu transferierende Proben markiert und somit ausgewählt werden.

Die Vorrichtung 1 weist zudem gemäß dem vorliegenden Ausführungsbeispiel eine ansteuerbare Isoliereinrichtung 6 auf, welche beispielsweise als geeigneter Laser, insbesondere als UVa-Laser, ausgebildet ist. Mittels der Isoliereinrichtung 6 wird nun die markierte und somit ausgewählte Probe bzw. ein vorbestimmter Bereich des Probenkörpers zum Isolieren der zu transferierenden Probe ausgeschnitten. Hierbei ist die Isoliereinrichtung 6 vorzugsweise mit einer Steuereinheit gekoppelt, welche insbesondere den Laserfokus, die Laserenergie und das Lasertriggern in Abhängigkeit bestimmter Vorgaben automatisch steuert.

Des Weiteren weist die Vorrichtung 1 beispielsweise eine ansteuerbare Transfereinrichtung 7 auf, welche in der europäischen Anmeldung mit der Anmeldenummer 08150662 exemplarisch näher beschrieben ist. Die in der europäische Patentanmeldung beschriebenen Merkmale der Transfereinrichtung sind daher von der vorliegenden Anmeldung mitumfasst.

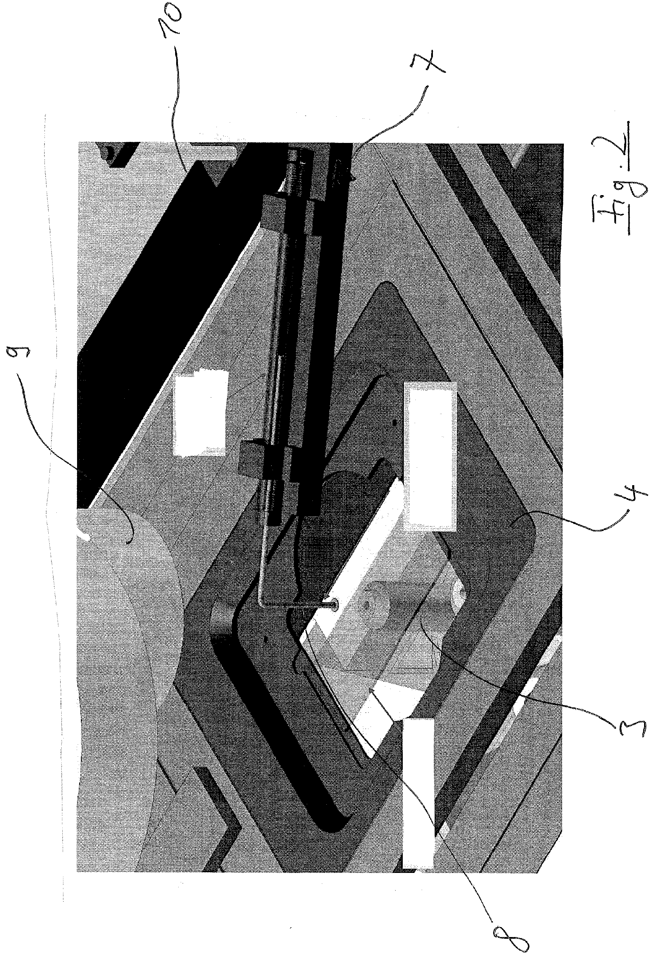

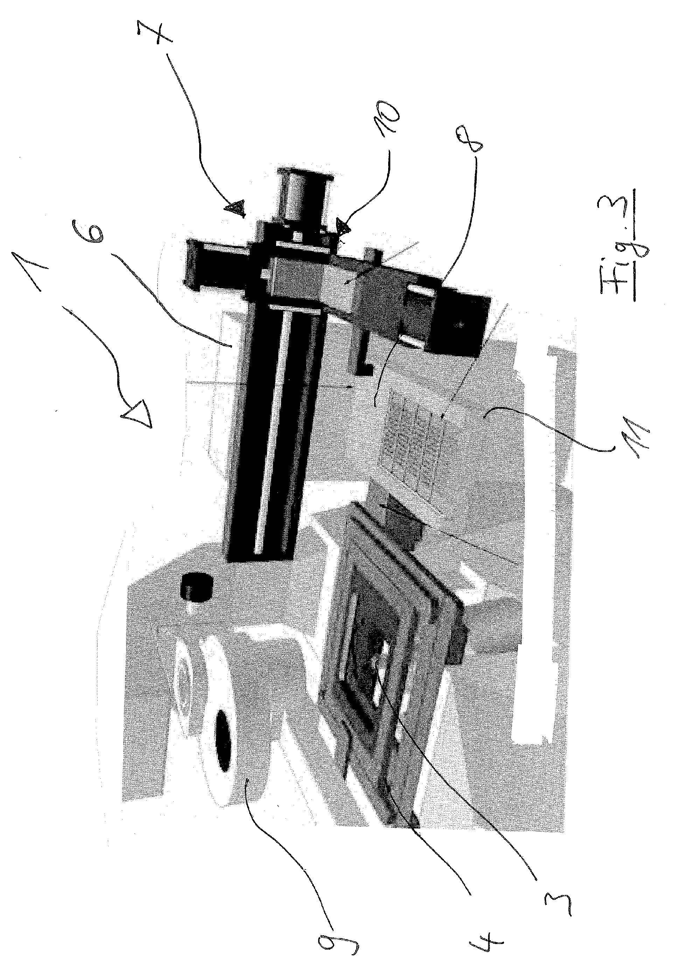

Die Transfereinrichtung 7 ist vorzugsweise autonom ausgebildet und in bestehende Systeme integrierbar, vorliegend in die Vorrichtung 1 derart integrierbar, dass die Transfereinrichtung 7 fest an der Mikroskopiereinheit 3 anbringbar ist. Die Transfereinrichtung weist beispielsweise eine Ansaugeinrichtung 8 auf, welche einem Ansaugen der Probe von dem Objekttisch 4 in der in Fig. 1 und in einer vergrößerten Ansicht in Fig. 2 dargestellten Probenentnahmeposition bzw. einem Abblasen der Probe in der in Fig. 3 dargestellten Probenabgabeposition dient.

Die Ansaugeinrichtung 8 weist beispielsweise einen Nanosauger auf, der beispielhaft aus einem Borsilikat-Glasröhrchen und einer Abschlussmembran besteht. Das Glasröhrchen besitzt gemäß dem vorliegenden Ausführungsbeispiel eine L-Form und ist an seinem einen Ende luftdicht mit einem Kopplungsröhrchen verbunden. Das andere Ende des Glasröhrchens weist stirnseitig eine Abschlussmembran auf, welche eine Trägerplatte und ein Abschlussgitter umfasst. Die Trägerplatte ist beispielhaft als Scheibe mit einem mittigen Loch ausgebildet. Die Trägerplatte besteht vorzugsweise aus einem elektrischleitfähigen Material, wie beispielsweise Kupfer oder dergleichen. Die Trägerplatte ist vorzugsweise derart an der Stirnseite des Kopfabschnitts des Nanosaugers befestigt, dass ein mittiges Loch der Trägerplatte mit der Öffnung des Saugrohrs fluchtet.

Die Abschlussmembran weist ferner ein Abschlussgitter auf, welches ebenfalls vorzugsweise aus einem elektrisch-leitfähigen Material oder einem Material mit elektrisch leitender Oberfläche ausgebildet ist. Das Abschlussgitter ist beispielsweise ebenfalls als runde Scheibe ausgebildet, wobei die Gitterstärke und die Porengröße des Abschlussgitters vorzugsweise an die jeweils zu transferierende Probe in geeigneter Weise angepasst werden kann. Vorstellbar ist beispielsweise eine Gitterstärke von in etwa 20 µm und Poren mit einem Durchmesser zwischen in etwa 60 nm und 50 µm. Das Abschlussgitter ist vorzugsweise derart auf der Trägerplatte aufgebracht, dass das Gitter das Loch der Trägerplatte und somit die Öffnung des Saugrohrs vollständig überdeckt. Die gesamte Anordnung ist vorzugsweise derart ausgebildet, dass die Saugluft direkt durch das Gitter und nicht an irgendwelchen undichten Stellen zur Umgebung geleitet wird.

Eine elektrostatische Aufladung des Nanosaugers ist zum Schutze der Probe zu verhindern. Daher sind für eine elektrische Verbindung zwischen der Trägerplatte und der elektrischleitfähigen Beschichtung des Saugrohrs elektrische Kontaktpunkte vorgesehen. Dadurch sollen elektrostatische Aufladungen, die beispielsweise durch bestimmte Proben oder das Material der Abschlussmembran hervorgerufen werden, vorteilhaft über das Saugrohr bzw. einer dem Saugrohr zugeordneten Erdungsleitung abgeleitet werden.

Des Weiteren umfasst die Transfereinrichtung 7 ferner eine Unter-/Überdruckeinheit, beispielsweise eine pneumatische Pico-Pumpe, welche den gewünschten Unterdruck zum Ansaugen der Probe auf das Abschlussgitter bzw. den jeweils gewünschten Überdruck zum Abblasen der Probe von dem Abschlussgitter auf das Auffangsystem 11 bereitstellt. Die Unter-/Überdruckeinheit ist zu diesem Zweck in geeigneter Weise mit der Ansaugeinrichtung 8 gekoppelt. Des Weiteren ist die Unter-/Überdruckeinheit mit der Steuereinheit datenverbunden, so dass eine entsprechend und dem jeweiligen Anwendungsfall angepasste Einstellung der Unter-/Überdruckeinheit gewährleistet ist. Insbesondere kann dadurch die Saugleistung bzw. die Stärke des Abblasimpulses der Unter-/Überdruckeinheit entsprechend dem jeweiligen Anwendungsfall vorzugsweise vorab bestimmt und entsprechend eingestellt und gesteuert werden. Die Regelbarkeit der Überdruckdauer von wenigen ms ist ebenfalls vorteilhaft, um die aufgenommene Probe sicher in einen vorgesehenen Flüssigkeitstropfen auf einer nachher noch zu erläuternden Analyseeinrichtung bzw. dem Auffangsystem 11 abgeben zu können, ohne diesen Tropfen zu zerstäuben.

Des Weiteren weist die Transfereinrichtung 7 vorzugsweise ein Autofokussystem auf, welches ebenfalls mit der Steuereinheit gekoppelt ist und die Annäherung des Unterdrucksaugsystems in einem definierten Abstand zur Probenoberfläche steuert. Beispielsweise wird ein geeigneter Lasersensor vorgesehen, welcher online den Abstand zwischen der Abschlussmembran und der aufzunehmenden Probe bzw. den Abstand zu dem Auffangsystem 11 misst, auf welche die aufgenommene Probe abzulegen ist. Beispielsweise kann als Lasersensor eine LTA-02 Lasersensor-Autofokuseinheit für die automatische Abstandsregelung verwendet werden, welche einen Sollabstand von zum Beispiel 100 µm zwischen den relevanten Oberflächen automatisch einstellt.

Die Transfereinrichtung 7 umfasst des Weiteren eine Trägereinrichtung 10, auf welcher zumindest die Ansaugeinrichtung 8 angeordnet ist. Die Trägereinrichtung 10 ist vorzugsweise mit einer zugeordneten Positioniereinheit gekoppelt. Vorzugsweise sind zur Verstellung der Trägereinrichtung 10 vier Freiheitsgrade der Positioniereinrichtung vorgesehen, drei translatorische Bewegungen sowie eine rotatorische Bewegung zum Verschwenken der Trägereinrichtung in horizontaler Ebene. Vorzugsweise werden schwingungsgedämpfte Schrittmotoren zur genauen Positionierung der Ansaugeinrichtung 8 verwendet. Allerdings ist es für einen Fachmann offensichtlich, dass hier auch andere Antriebe verwendet werden können, die entsprechend geeignet sind. Beispielsweise wird ein Mikrometer-Schrittmotor verwendet, welcher die Trägereinrichtung 10 und somit den Nanosauger mit Genauigkeit im µm-Bereich positionieren kann.

Im Falle einer Anbringung der Transfereinrichtung 7 an einer zugeordneten Mikroskopiereinheit 3 muss lediglich der Objekttisch 4 entsprechend verstellt werden, wobei bei einer Verschwenkung der Trägereinrichtung 10 eine exakte Positionierung des Nanosaugers über dem Linsensystem bzw. dem Lasersystem der Mikroskopiereinheit 3 gewährleistet ist, da aufgrund der festen Anbringung die Verstellung der Trägereinrichtung 10 in dem Bezugssystem der Mikroskopiereinheit 3 erfolgt. Dadurch wird eine maximale Genauigkeit und ein schnelles Aufnehmen der jeweiligen Proben, auch noch so kleiner Proben, gewährleistet. Die gesamte Verstellbewegung wird mittels einer entsprechenden Software und der oben bereits genannten zentralen Steuereinheit automatisiert ausgeführt.

Die Vorrichtung 1 umfasst ferner ein Auffangsystem 11, welches vorzugsweise in einem geschützten Schlittenschacht untergebracht ist und als Ablage der einzelnen Proben dient.

Das Auffangsystem besteht beispielsweise aus einem strukturierten Aufnahmeträger mit einzelnen Ablagepunkte unter einer geeigneten Abdeckung, wobei lediglich ein vorbestimmter Öffnungsbereich zur Aufnahme der entsprechenden Probe bzw. Proben durch die Transfereinrichtung 7 gewährleistet ist. Nur ein kleiner Bereich ist für die Transfereinrichtung 7 zugänglich, um mögliche externe Kontamination der zu transferierenden und zu analysierenden Probe/n zu verhindern. Auch das Auffangsystem 11 ist vorzugsweise mittels eines entsprechenden Adapters an eine vorgegebene Mikroskopiereinheit 3 fest anbringbar.

Die einzelnen oder zumindest eine bestimmte Auswahl der oben genannten Bauteile sind vorzugsweise mit der zentralen Steuereinheit datenverbunden derart, dass mittels der zentralen Steuereinheit durch Auswahl der zu isolierenden und zu transferierenden Probe/n der gesamte Isolier- und Transferierungssowie Ablegevorgang auf einem strukturierten Auffangsystem vollständig automatisiert ablaufen kann. Ferner werden durch die oben beschriebene Vorrichtung 1 eine definierte und kontaminationsfreie Isolierung und Transferierung gewährleistet. Alle Prozessschritte mittels der oben genannten Vorrichtung 1 laufen synchronisiert und vollständig automatisiert ab, so dass eine Hochdurchsatz-Analytik gewährleistet wird. Das Unterdrucksaugsystem ermöglicht die kontaktfreie Aufnahme der isolierten Proben vom Probenträger und die gezielte Ablage in kleinste Volumenmengen, beispielsweise 50 bis 400 nl, bevorzugt maximal 1000 nl, auf jede Art von strukturiertem Auffangsystem. Zum Beispiel kann es sich bei dem Auffangsystem um eine Multititerplatte, einen funktionalisierten Glasobjektträger oder einen Biochip (Lab-on-a-chip System) handeln. Dabei können die Proben einzeln oder gepoolt aufgenommen werden. Hierzu wird der an die Transfereinrichtung 7 angelegte Unterdruck aufrechterhalten, wobei als Ansaugeinrichtung 8 ein oder mehrere Sauger zur Probenaufnahme verwendet werden können. Diese Sauger können dabei einzeln oder zusammen mittels der zentralen Steuereinheit angesteuert werden. Über dem vorbestimmten Ablageort wird die isolierte Probe oder die isolierten Proben gezielt in einen kleinen Flüssigkeitstropfen zur weiteren Analyse abgelegt.

Die weitere Analyse umfasst beispielsweise eine biochemische Analyse, eine optische Analyse, eine PCR-Analyse, eine Nukleinsäure Analyse und/oder eine Protein Analyse.

Die Vorrichtung 1 kann auch weitere Komponenten neben den oben aufgeführten aufweisen, beispielsweise eine Feed-Back-Kontrolleinrichtung (nicht dargestellt), welche dem Benutzer auf einfache Weise eine Überprüfung gewährleistet, ob eine Probe wunschgemäß in dem Auffangsystem abgelegt wurde oder nicht. Die Feed-Back-Kontrolleinrichtung weist beispielsweise Bragg-Spiegel auf. Diese Bragg-Spiegel können als Gitter angesehen werden und verändern im Falle einer positiven Ablage einer Probe messbar deren Reflektion derart, dass ein positives Rückkopplungssystem zum Bestätigen einer positiven Ablage geschaffen werden kann. Dies ist vorteilhaft, da weitere, nachgeschaltete biochemische Prozesse nur durchgeführt werden können, wenn es vorher zu einer positiven Ablage gekommen ist.

Die Steuerung erfolgt vorzugsweise über eine speziell entwickelte Software und ermöglicht unter anderem die notwendige Regelung der Überdruckdauer von wenigen ms sowie die Schwenkbewegung der Transfereinrichtung, um die aufgenommene Probe sicher in beispielsweise einen vorgegebenen Flüssigkeitstropfen des Auffangsystems 11 abgeben zu können, ohne ihn zu zerstäuben. Alle für die Steuerung notwendigen Parameter können über die Software frei eingestellt werden.

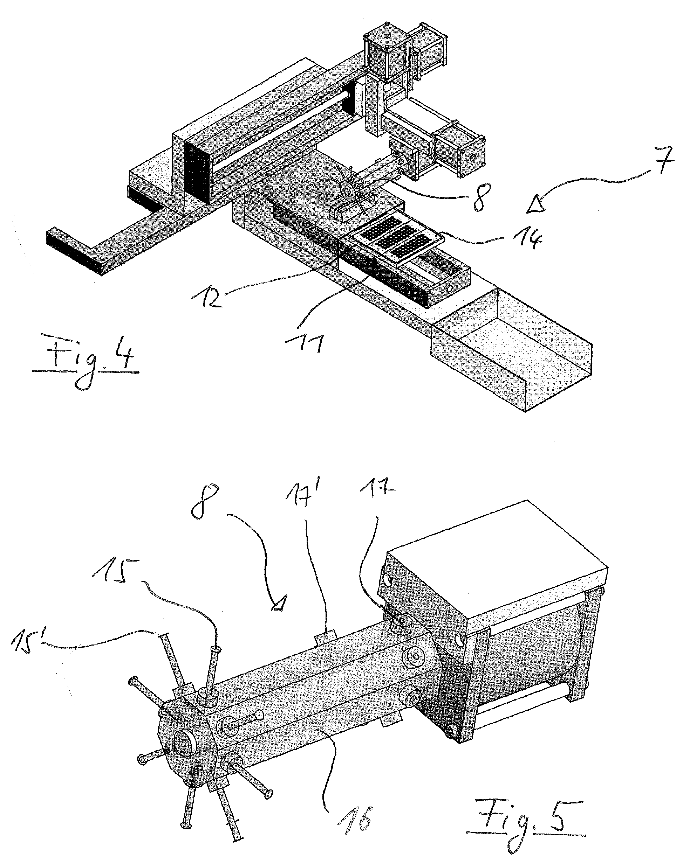

In den Fig. 4 und 5 ist eine Transfereinrichtung 7 gemäß einem zweiten bevorzugten Ausführungsbeispiel der vorliegenden Erfindung dargestellt und wird im Folgenden näher erläutert. Hinsichtlich im Folgenden nicht näher beschriebenen Komponenten, Ausgestaltungen und Wirkungsweisen der einzelnen Komponenten wird auf das oben beschriebene Ausführungsbeispiel verwiesen.

Zunächst einmal ist in Fig. 4 ersichtlich, dass das Auffangsystem 11 aus einem freilegbaren Schlittenschacht 12 und einem klappbaren Deckel 13 besteht. Die einzelnen Bereiche der Ablegeplatte 14 können über das Schlittensystem in und aus einem geschützten Schachtbereich hinein- und herausgefahren werden. Dies dient einer Verhinderung möglicher externer Kontamination der abgelegten Proben. Das in Fig. 4 dargestellte Auffangsystem ist selbstverständlich auch auf das erste Ausführungsbeispiel gemäß den Fig. 1-3 übertragbar.

Des Weiteren ist in Fig. 5 eine vergrößerte Darstellung der Ansaugeinrichtung 8 gemäß dem zweiten bevorzugten Ausführungsbeispiel aus Fig. 4 dargestellt. Diese Ansaugeinrichtung 8 dient einer Aufnahme mehrerer, gemäß dem vorliegenden Ausführungsbeispiel von maximal acht, Proben mittels einzelner Sauger 15, 15'.

Die Ansaugeinrichtung 8 gemäß dem Ausführungsbeispiel nach Fig. 5 weist beispielsweise einen drehbaren Zylinder bzw. Revolver 16 auf, wobei die einzelnen Sauger 15, 15' über einzelne Ansaugrohre verfügen und diese Ansaugrohre wiederum einzeln über jeweilige Druckleitungen ansteuerbar sind. Die einzelnen Druckleitungen werden an die den jeweiligen Saugern 15, 15' zugeordneten Anschlüssen 17, 17' entsprechend luftdicht angeschlossen und mittels der zentralen Steuereinheit entsprechend angesteuert. Dadurch wird ein individuelles Ansaugen bzw. Ablegen der jeweiligen isolierten Proben ermöglicht. Die einzelnen Verbindungen der Druckleitungen zu den Anschlüssen 17, 17' erfolgt beispielsweise mittels eines "Easy-to-Click" Systems, so dass die jeweils zu verwendende Anzahl an Saugern auf einfache Weise frei gewählt werden kann. Im Falle der Verwendung lediglich eines Saugers 15 ist keine Rotation des Zylinders bzw. Revolvers 16 notwendig. Bei einer Verwendung von mehreren Saugern 15, 15' werden zusätzliche Druckleitungen an die entsprechenden Anschlüsse 17, 17' angelegt, der Zylinder bzw. Revolver 16 entsprechend gedreht und die einzelnen isolierten Proben nacheinander aufgenommen. Des Weiteren ist gemäß dem vorliegenden Ausführungsbeispiel ein einfacher Austausch der einzelnen Sauger 15, 15' gewährleistet, was zu einem kontaminationsfreien Vorgang beiträgt.

Die Ansaugeinrichtung 8 bzw. die einzelnen Sauger 15 können dabei mittels der zentralen Steuereinheit einzeln ausgewählt und derart angesteuert werden, dass ein bestimmter Sauger 15 mittels verdrehen des Zylinders 16 der Ansaugeinrichtung 8 über den Objekttisch 4 verfahren wird und eine isolierte Probe aufnimmt. Nach Aufnahme dieser Probe durch diesen Sauger 15 wird der Zylinder 16 der Ansaugeinrichtung 8 um einen vorbestimmten Winkel derart gedreht, dass ein weiterer vorbestimmter Sauger 15' über dem Objekttisch 4 zur Aufnahme einer weiteren, bereits isolierten Probe steht. Entsprechend der Anzahl und Reihenfolge der anfangs ausgewählten, zu isolierenden und zu transferierenden Proben wird dieser Schritt sukzessive wiederholt, maximal bis alle Sauger 15, 15' eine entsprechende isolierte Probe aufgenommen haben. Anschließend wird die Ansaugeinrichtung 8 mittels der Transfereinrichtung 7 zu dem Auffangsystem 11 mittels der vorgesehenen Schrittmotoren verstellt. Die einzelnen Sauger 15, 15' werden anschließend sukzessive über den entsprechenden Ablagepunkten der Ablegeplatte 14 des Auffangsystems 11 positioniert mittels der Steuereinheit und die daran anhaftenden Proben entsprechend abgelegt, wobei der Zylinder bzw. Revolver 16 nach Ablage einer Probe wieder entsprechend derart gedreht wird, dass eine weitere Probe durch das Unter-/Überdrucksystem in geeigneter Weise abgelegt werden kann.

Somit schafft die vorliegende Erfindung eine Vorrichtung und ein Verfahren zum automatisierten Isolieren und Transferieren von einer oder mehreren mikroskopischen Proben von einem Probenträger zu einem Auffangsystem mittels Trägermitteln. Vorteilhaft wird der horizontale Transport und eine gezielte Ablage von mikrodissektierten Proben auf strukturierten Ablagen in einem Flüssigkeitstropfen ermöglicht. Dabei erfolgt das Isolieren und Transportieren der mikrodissektierten Proben vollständig automatisiert, wobei zu jedem Zeitpunkt des Vorgangs volle Kontrolle über die einzelnen Arbeitsschritte bzw. der Zustand der jeweils isolierten Probe/n besteht. Des Weiteren ist die oben beschriebene Vorrichtung auf einfache Weise bedienbar, da lediglich über den Touch-Screen eine Auswahl der zu analysierenden Proben erfolgen muss.

Obwohl die vorliegende Erfindung anhand bevorzugter Ausführungsbeispiele vorliegend beschrieben wurde, ist sie darauf nicht beschränkt, sondern auf vielfältige Weise modifizierbar. Insbesondere ist die Ansaugeinrichtung auch andersartig als oben erläutert ausführbar, solange eine vollautomatisierte Aufnahme und Ablage einer oder mehrerer Proben gewährleistet wird. Ferner ist beispielsweise das Auffangsystem bzw. die darin integrierte Ablegeplatte beliebig skalierbar aufgrund der hohen Genauigkeit und Präzision der Transfereinrichtung. Dadurch können vorteilhaft sowohl Mikrotiterplatten wie auch Mikrochips mit Nanoliter-Tröpfchen angesteuert werden.

### Bezugszeichenliste

- 1: Vorrichtung
- 2: Auswähleinrichtung
- 3: Mikroskopiereinheit
- 4: Probenträger/Objekttisch
- 5: Anzeigeeinrichtung
- 6: Isoliereinrichtung
- 7: Transfereinrichtung
- 8: Ansaugeinrichtung
- 9: Kondensor
- 10: Trägereinrichtung
- 11: Auffangsystem
- 12: Schlittenschacht
- 13: Deckel
- 14: Ablegeplatte
- 15, 15': Sauger
- 16: Zylinder
- 17, 17': Anschlüsse

## Patentansprüche

1. Vorrichtung (1) zum automatisierten Isolieren und Transferieren mindestens einer mikroskopischen Probe von einem Probenträger (4) zu einem Auffangsystem (11) für eine automatische, nachgelagerte Analyse, mit:
einer Auswähleinrichtung (2) zum Auswählen mindestens einer sich auf dem Probenträger (4) befindlichen Probe;
einer ansteuerbaren Isoliereinrichtung (6) zum automatisierten Isolieren der mindestens einen ausgewählten Probe;
einer ansteuerbaren Transfereinrichtung (7), welche Trägermittel zum definierten Aufnehmen und definierten Ablegen der mindestens einen ausgewählten, isolierten Probe für einen automatisierten und definierten Transfer der mindestens einen ausgewählten, isolierten Probe von dem Probenträger (4) zu dem Auffangsystem (11) aufweist; und
einer Steuereinheit, welche für einen automatisierten Isolier- und Transfervorgang mit der Auswähleinrichtung (2), der Isoliereinrichtung (6) und der Transfereinrichtung (7) datenverbunden ist.

2. Vorrichtung nach Anspruch 1,
**dadurch gekennzeichnet,**
**dass** die Auswähleinrichtung (2) eine Mikroskopiereinheit (3) und eine damit gekoppelte Anzeigeeinrichtung (5), insbesondere einen Touch-Screen (5), zum Darstellen des mikroskopischen Bildes der auf dem Probenträger (4) zur Verfügung stehenden Proben aufweist.

3. Vorrichtung nach Anspruch 2,
**dadurch gekennzeichnet,**
**dass** die Anzeigeeinrichtung (5) Auswählmittel zum Auswählen der mindestens einen zu isolierenden und zu transferierenden Probe aufweist, insbesondere unter Zuhilfenahme optischer Markierungsmittel zum graphischen Kennzeichnen der mindestens einen zu isolierenden und zu transferierenden Probe auf dem Touch-Screen.

4. Vorrichtung nach einem der vorhergehenden Ansprüche, **dadurch gekennzeichnet,**
**dass** die Isoliereinrichtung (6) einen geeignet ausgestalteten Laser, insbesondere einen UVa-Laser, zum automatisierten und kontaminationsfreien Isolieren der mindestens einen ausgewählten Probe aufweist.

5. Vorrichtung nach einem der vorhergehenden Ansprüche, **dadurch gekennzeichnet,**
**dass** die Transfereinrichtung (7) ein geeignet ausgestaltetes Unterdrucksaugsystem (8) zum automatisierten, definierten und kontaminationsfreien Transferieren der mindestens einen zu transferierenden Probe aufweist.

6. Vorrichtung nach Anspruch 5,
**dadurch gekennzeichnet,**
**dass** die Transfereinrichtung (7) ein Autofokussystem zum positionsgenauen Einstellen des Unterdrucksaugsystems (8) in einem vorbestimmten Abstand zu der mindestens einen zu transferierenden Probe aufweist.

7. Vorrichtung nach einem der vorhergehenden Ansprüche,
**dadurch gekennzeichnet,**
**dass** das Unterdrucksaugsystem (8) mehrere, einzeln ansteuerbare Saugeinrichtungen (15, 15') zum gleichzeitigen Transferieren mehrerer Proben aufweist.

8. Vorrichtung nach einem der vorhergehenden Ansprüche,
**dadurch gekennzeichnet,**
**dass** das Auffangsystem (11) einen teilweise geschützten Abschnitt, insbesondere einen teilweise geschützten Schlittenschacht (12), aufweist, wobei ein angepasster Öffnungsbereich für ein Ablegen der mindestens einen Probe auf dem Auffangsystem (11) durch die Transfereinrichtung (7) vorgesehen ist.

9. Vorrichtung nach einem der vorhergehenden Ansprüche,
**dadurch gekennzeichnet,**
**dass** ferner eine Feed-Back-Kontrolleinrichtung zum Bestätigen einer erfolgreichen Ablage der mindestens einen Probe auf dem Auffangsystem (11) vorgesehen ist, welche beispielsweise Bragg-Spiegel derart aufweist, dass sich deren Reflektion nach positiver Ablage der mindestens einen Probe messbar ändert.

10. Verfahren zum automatisierten Isolieren und Transferieren mindestens einer mikroskopischen Probe von einem Probenträger (4) zu einem Auffangsystem (11), mit folgenden Verfahrensschritten:
Auswählen mindestens einer sich auf dem Probenträger (4) befindlichen Probe mittels einer Auswähleinrichtung (2);
automatisiertes Isolieren der mindestens einen ausgewählten Probe mittels einer ansteuerbaren Isoliereinrichtung (6);
automatisiertes und definiertes Transferieren der mindestens einen ausgewählten, isolierten Probe von dem Probenträger (4) zu dem Auffangsystem (11) mittels einer ansteuerbaren Transfereinrichtung (7), welche Trägermittel zum definierten Aufnehmen und definierten Ablegen der mindestens einen ausgewählten, isolierten Probe aufweist; und
Ansteuern der Isoliereinrichtung (6) und der Transfereinrichtung (7) mittels einer mit der Auswähleinrichtung (2), der Isoliereinrichtung (6) und der Transfereinrichtung (7) datenverbundenen Steuereinheit in Abhängigkeit des Schrittes des Auswählens der mindestens einen Probe.

11. Verfahren nach Anspruch 10,
**dadurch gekennzeichnet,**
**dass** ein mikroskopisches Bild der auf dem Probenträger (4) zur Verfügung stehenden Proben mittels einer Mikroskopiereinheit (3) und einer damit gekoppelten Anzeigeeinrichtung, insbesondere einem Touch-Screen, dargestellt wird.

12. Verfahren nach Anspruch 11,
**dadurch gekennzeichnet,**
**dass** die mindestens eine zu isolierende und zu transferierende Probe mittels Auswählmitteln ausgewählt wird, insbesondere mittels optischen Markierungsmitteln zum graphischen Kennzeichnen der mindestens einen zu isolierenden und zu transferierenden Probe auf dem Touch-Screen.

13. Verfahren nach einem der Ansprüche 10 bis 12,
**dadurch gekennzeichnet,**
**dass** die mindestens eine ausgewählte Probe mittels eines geeignet ausgestalteten Lasers (6), insbesondere eines UVa-Lasers, automatisiert, definiert und kontaminationsfrei isoliert wird.

14. Verfahren nach einem der Ansprüche 10 bis 13,
**dadurch gekennzeichnet,**
**dass** die mindestens eine zu transferierende Probe mittels eines geeignet ausgestalteten Unterdrucksaugsystems (8) automatisiert, definiert und kontaminationsfrei transferiert wird.

15. Verfahren nach Anspruch 14,
**dadurch gekennzeichnet,**
**dass** das Unterdrucksaugsystem (8) zum positionsgenauen Einstellen desselben in einem vorbestimmten Abstand zu der mindestens einen zu transferierenden Probe mittels eines Autofokussystem verstellt wird.

16. Verfahren nach einem der Ansprüche 14 oder 15,
**dadurch gekennzeichnet,**
**dass** das Unterdrucksaugsystem (8) mit mehreren, einzeln ansteuerbaren Ansaugeinrichtungen (8; 15, 15') zum gleichzeitigen Transfer mehrerer Proben ausgebildet wird.

17. Verfahren nach einem der Ansprüche 10 bis 16,
**dadurch gekennzeichnet,**
**dass** das Auffangsystem (11) mit einem teilweise geschützten Abschnitt ausgebildet wird, insbesondere mit einem teilweise geschützten Schlittenschacht (12), wobei ein angepasster Öffnungsbereich für ein Ablegen der mindestens einen Probe auf dem Auffangsystem (11) durch die Transfereinrichtung (7) vorgesehen wird.

18. Verfahren nach einem der Ansprüche 10 bis 17,
**dadurch gekennzeichnet,**
**dass** eine erfolgreiche Ablage der mindestens einen Probe auf dem Auffangsystem (11) mittels einer Feed-Back-Kontrolleinrichtung durchgeführt wird, welche beispielsweise Bragg-Spiegel derart aufweist, dass sich deren Reflektion nach positiver Ablage der mindestens einen Probe messbar ändert.

19. Verfahren nach einem der Ansprüche 10 bis 18,
**dadurch gekennzeichnet,**
**dass** die Probe nach einer erfolgereichen Ablage derselben auf dem Auffangsystem (11) automatisch analysiert wird, wobei die Analyse beispielsweise eine biochemische Analyse, eine optische Analyse, eine PCR-Analyse, eine Nukleinsäure Analyse und/oder eine Protein Analyse ist.
